# FASCICULE DE BREVET EUROPEEN

(11) **EP 3 762 119 B1**
(45) Date de publication et mention de la délivrance du brevet: **06.12.2023**
(21) Numéro de dépôt: 19715161.6
(22) Date de dépôt: 07.03.2019
(51) Int. Cl.: B01D 15/18, B01D 15/36, B01J 41/20, B01J 41/05

(54) **SEPARATION CHROMATOGRAPHIQUE DU SULFATE D'AMMONIUM ET DE L'ACIDE 2-HYDROXY-2-METHYLPROPIONIQUE**
CHROMATOGRAFISCHE TRENNUNG VON AMMONIUMSULFAT UND 2-HYDROXY-2-METHYLPROPIONSÄURE
CHROMATOGRAPHIC SEPARATION OF AMMONIUM SULPHATE AND OF 2-HYDROXY-2-METHYLPROPIONIC ACID

(30) Priorité: 09.03.2018 FR 1852067
(43) Date de publication de la demande: 13.01.2021
(73) Titulaire: Trinseo Europe GmbH, 8808 Pfaeffikon (CH)
(72) Inventeur: LESAFFRE, Thibault, 69300 Caluire et Cuire (FR); EDON, Marjory, 69220 CORCELLES-EN BEAUJOLAIS (FR); REDELSPERGER, Laurine, 01800 BOURG SAINT CHRISTOPHE (FR); DUPLESSIX, Aymeric, 69003 Lyon (FR)
(74) Mandataire: SSM Sandmair
(86) Numéro de dépôt international: PCT/FR2019/050506
(87) Numéro de publication internationale: WO 2019/171004

(56) Documents cités:
- CA-A- 486 040
- GB-A- 578 307
- US-A- 4 981 794
- US-A1- 2012 329 096

## Description

### DOMAINE DE L'INVENTION

La présente invention concerne un procédé de purification ou de séparation par chromatographie d'un flux de départ contenant du sulfate d'ammonium (et éventuellement d'autres sels) et de l'acide 2-hydroxy-2-méthylpropionique (et éventuellement d'autres composés organiques). Ce procédé de purification est particulièrement utile dans le cadre du traitement des effluents issus de la production du méthacrylate de méthyle à partir de cyanhydrine d'acétone.

### ARRIERE-PLAN TECHNIQUE

Le méthacrylate de méthyle (MMA) est couramment utilisé notamment pour la production de verre organique en polyméthacrylate de méthyle (PMMA). Ce monomère permet de conférer stabilité, résistance, dureté et brillance aux polymères dont il entre dans la composition.

Une voie classique de synthèse du MMA est décrite dans l'ouvrage Encyclopedia of Chemical Technology, Kirk-Othme, 4ème édition, volume 16, p.244-260 (2004). Elle repose sur l'utilisation de cyanhydrine d'acétone (ACH) en tant que matériau de départ.

Dans le cadre de cette voie de synthèse, des quantités importantes d'effluents contenant du sulfate d'ammonium peuvent être générées.

L'article Improvement of Industrial Synthesis of Methyl Methacrylate, Application of a Wet Air Oxidation Process, par Dario Giudici, dans La Chimica e L'industria, 82:873-878 (2000), décrit un traitement d'un tel effluent par cristallisation. La purge de la cristallisation, contenant du sulfate d'ammonium et des espèces non ionisées, parmi lesquelles l'acide 2-hydroxy-2-méthylpropionique, est ensuite traitée par un procédé d'oxydation à l'air humide (WAO). Celui-ci est mis en oeuvre dans des conditions drastiques de pression et température, en raison de la forte teneur en sel.

Le document US 4,015,946 décrit une séparation par décantation d'une phase aqueuse constituée du sulfate d'ammonium et d'une phase constituée de composés organiques. Cette séparation est possible lorsque la solution à traiter est saturée en sulfate d'ammonium, et elle repose sur l'ajout d'un lactame. Ce procédé a pour inconvénient de consommer un composé organique supplémentaire, et il n'est applicable que pour certaines concentrations de sulfate d'ammonium.

Les documents CA 486 040 A et GB 578 307 A décrivent la purification d'acide 2-hydroxy-2-méthylpropionique par passage d'une solution en contenant, ainsi que du sulfate d'ammonium, sur une résine échangeuse d'anions. Dans ces procédés, le sulfate d'ammonium n'est pas récupéré. Il existe donc un besoin de fournir un traitement d'un effluent contenant du sulfate d'ammonium et de l'acide 2-hydroxy-2-méthylpropionique (et éventuellement d'autres composés organiques), et ne présentant pas les inconvénients ci-dessus.

### RESUME DE L'INVENTION

L'invention concerne en premier lieu un procédé de purification chromatographique d'un flux de départ contenant du sulfate d'ammonium et de l'acide 2-hydroxy-2-méthylpropionique, comprenant :
- le passage du flux de départ sur un lit de phase stationnaire ;
- l'élution d'un raffinat enrichi en sulfate d'ammonium et appauvri en acide 2-hydroxy-2-méthylpropionique ; et
- l'élution d'un extrait enrichi en acide 2-hydroxy-2-méthylpropionique et appauvri en sulfate d'ammonium.

Dans certains modes de réalisation, la purification est effectuée par chromatographie d'exclusion d'ions.

Dans certains modes de réalisation, la phase stationnaire est une résine cationique, de préférence une résine cationique forte, et de préférence encore une résine cationique forte sous forme de contre-ion NH₄⁺.

Dans certains modes de réalisation, la phase stationnaire est constituée de particules ayant une taille Dv50 comprise entre 200 et 350 µm.

Dans certains modes de réalisation, la phase stationnaire est préalablement équilibrée avec une solution contenant des ions ammonium, et de préférence avec le flux de départ.

Dans certains modes de réalisation, le flux de départ est à un pH de 1 à 4 lors de son passage sur le lit de phase stationnaire.

Dans certains modes de réalisation, on fait passer un éluant sur le lit de phase stationnaire pour l'élution du raffinat et l'élution de l'extrait, ledit éluant étant de préférence de l'eau déminéralisée ou de l'eau déminéralisée acidifiée, notamment à un pH de 3 à 5, l'eau déminéralisée acidifiée ayant de préférence une teneur en acide de 0,0005 à 5 g/L, de préférence encore de 0,005 à 0,02 g/L.

Dans certains modes de réalisation, le procédé est mis en oeuvre à une température de 20 à 85°C, de préférence de 50 à 80°C.

Dans certains modes de réalisation, le procédé est mis en oeuvre sur un système chromatographique multicolonnes, de préférence un système à lit mobile simulé, et de préférence encore un système à lit mobile simulé séquentiel.

Dans certains modes de réalisation, le système chromatographique comprend une zone 4 située entre une ligne de collecte de raffinat et une ligne d'injection d'éluant, la zone 4 étant traversée par un volume de phase mobile compris entre 0,3 et 0,55 BV, de préférence entre 0,4 et 0,5 BV.

Dans certains modes de réalisation, le système chromatographique multicolonnes comprend moins de 12 colonnes, ou moins de 8 colonnes, et de préférence de 4 à 6 colonnes.

Dans certains modes de réalisation, le système chromatographique multicolonnes comprend des colonnes ayant un diamètre supérieur ou égal à 2 mètres.

Dans certains modes de réalisation, le système chromatographique multicolonnes comprend des colonnes ayant une hauteur comprise entre 1 et 2,5 mètres.

Dans certains modes de réalisation, le procédé est mis en oeuvre avec une vitesse opératoire moyenne d'élution supérieure à 1 m/h, ou supérieure à 2 m/h, ou supérieure à 4 m/h, et préférablement supérieure à 5 m/h.

Dans certains modes de réalisation, le flux de départ est issu d'un procédé de production de méthacrylate de méthyle, ledit procédé de production de méthacrylate de méthyle comprenant de préférence :
- la préparation de sulfate de méthacrylamide par réaction de la cyanhydrine d'acétone avec de l'acide sulfurique ;
- la préparation de méthacrylate de méthyle par réaction dudit sulfate de méthacrylamide avec un mélange d'eau et de méthanol ;
- la collecte d'un effluent acide ;
- la neutralisation de l'effluent acide avec de l'ammoniac ;
- la cristallisation de l'effluent acide neutralisé pour obtenir d'une part des cristaux de sulfate d'ammonium et d'autre part une purge de cristallisation, cette purge de cristallisation fournissant ledit flux de départ.

La présente invention permet de surmonter les inconvénients de l'état de la technique. Elle fournit plus particulièrement un traitement d'un effluent contenant du sulfate d'ammonium et de l'acide 2-hydroxy-2-méthylpropionique et éventuellement d'autres composés organiques, permettant de recycler les sels vers une étape de cristallisation par exemple, et de renvoyer les composés organiques vers une étape d'oxydation par exemple, comme proposé dans l'article de D. Giudici.

Le procédé de l'invention ne consomme pas de composé organique supplémentaire. Il peut également être mis en oeuvre avec diverses concentrations en sulfate d'ammonium. Aucun minimum de concentration n'est prérequis.

Le procédé de l'invention peut ainsi permettre d'abaisser la teneur en sel dans le flux à traiter, et donc d'employer des matériaux moins résistants aux sels et à l'acidité.

### BREVE DESCRIPTION DES FIGURES

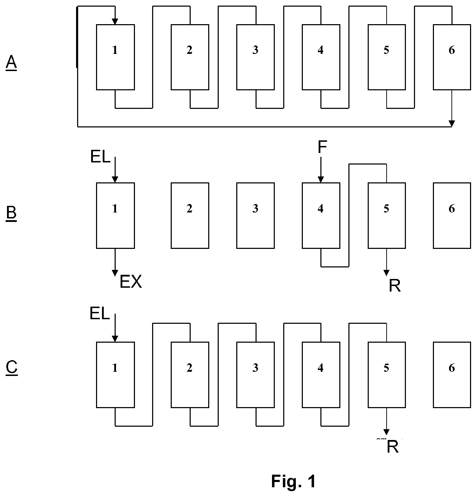

La **figure 1** représente de manière schématique un système chromatographique SSMB susceptible d'être utilisé pour mettre en oeuvre le procédé de l'invention.

### DESCRIPTION DE MODES DE REALISATION DE L'INVENTION

L'invention est maintenant décrite plus en détail et de façon non limitative dans la description qui suit.

### Production de MMA

L'invention est appliquée de préférence dans le cadre de la synthèse du MMA à partir de l'ACH.

Cette synthèse, décrite dans l'ouvrage de Kirke-Othme, comprend de préférence les deux étapes principales suivantes :
- réaction de l'ACH avec de l'acide sulfurique pour former du sulfate de méthacrylamide ;
- estérification du sulfate de méthacrylamide en présence d'un mélange d'eau et de méthanol, pour former du MMA et un effluent acide.

La première étape peut comporter deux sous-étapes : d'abord la conversion de l'ACH en un intermédiaire, le monoester sulfurique de 2-hydroxy-2-méthylpropionamide, de préférence à basse température ; puis la formation du sulfate de méthacrylamide à partir de cet intermédiaire, de préférence à température élevée (par exemple de 100 à 140°C).

Une partie de l'ACH forme toutefois divers produits secondaires.

A l'issue de la deuxième étape d'estérification, comme décrit dans l'article de D. Giudici, on récupère le MMA souhaité, ainsi qu'un effluent acide, qui est une solution aqueuse contenant du bisulfate d'ammonium, de l'acide sulfurique, et divers composés organiques.

Le flux de départ de la purification chromatographique de l'invention est avantageusement obtenu à partir de cet effluent acide.

Par exemple, comme décrit dans l'article de D. Giudici, l'effluent acide peut être neutralisé avec de l'ammoniac, ce qui conduit à la formation de sulfate d'ammonium. Cet effluent acide neutralisé peut former le flux de départ.

Alternativement et de préférence, comme décrit dans l'article de D. Giudici, une cristallisation est effectuée à partir de l'effluent acide neutralisé, au moyen d'un cristalliseur, pour récupérer des cristaux de sulfate d'ammonium. Une telle cristallisation fournit des cristaux d'une part, de la vapeur d'autre part, et enfin une phase liquide, les eaux-mères. Une partie des eaux-mères est recyclée vers le cristalliseur, tandis que l'autre partie est éliminée, cette dernière étant désignée « *purge de cristallisation* ». La purge de cristallisation est une solution aqueuse contenant notamment du sulfate d'ammonium et de l'acide 2-hydroxy-2-méthylpropionique (et éventuellement d'autres composés organiques).

Le flux de départ de la purification chromatographique de l'invention peut être directement constitué par la purge de cristallisation. Alternativement, le flux de départ peut être obtenu par exemple en diluant la purge de cristallisation, et/ou en y ajoutant un ou des produits.

Par exemple, il peut être utile d'ajuster le pH du flux de départ avant de le soumettre à la purification chromatographique, par ajout d'un ou plusieurs acides ou bases.

### Purification chromatographique

L'invention prévoit de traiter le flux de départ avec les étapes suivantes :
- le passage du flux de départ sur un lit de phase stationnaire (étape de charge) ;
- l'élution d'un raffinat enrichi en sulfate d'ammonium (et appauvri en acide 2-hydroxy-2-méthylpropionique) ; et
- l'élution d'un extrait enrichi en acide 2-hydroxy-2-méthylpropionique (et appauvri en sulfate d'ammonium).

Plus généralement, le raffinat est enrichi en espèces ionisées et appauvri en espèces non ionisées ; et l'extrait est enrichi en espèces non ionisées (ou neutres) et appauvri en espèces ionisées.

Par « *espèces ionisées* » on entend les espèces se trouvant dans le flux de départ sous forme ionisée dans les conditions de température et de pH mises en oeuvre pour la purification chromatographique. Dans l'invention, les espèces ionisées peuvent comprendre notamment des sels.

Par « *espèces non ionisées* » on entend les espèces se trouvant dans le flux de départ sous forme non ionisée dans les conditions de température et de pH mises en oeuvre pour la purification chromatographique. Dans l'invention les espèces non ionisées peuvent comprendre notamment des acides organiques.

Par « *raffinat* », on entend la fraction obtenue par élution qui contient les espèces relativement les moins retenues par la phase stationnaire, et donc dont l'élution est la plus rapide.

Par « *extrait »,* on entend la fraction obtenue par élution qui contient les espèces relativement les plus retenues par la phase stationnaire, et donc dont l'élution est la plus lente.

Par fraction « *enrichie »* en une espèce A et « *appauvrie »* en une espèce B, on entend que le rapport de concentrations molaires espèce A / espèce B dans la fraction est supérieur à celui du flux de départ (indépendamment des effets de concentration ou de dilution globale).

Comme décrit dans l'article de D. Giudici, le flux de départ contient du sulfate d'ammonium et de l'acide 2-hydroxy-2-méthylpropionique. De préférence, il contient également d'autres composés organiques qui constituent des espèces non ionisées, et qui peuvent être également collectées dans l'extrait. Ces espèces non ionisées comprennent par exemple des oligomères du MMA, et divers composés organiques sulfonés. Le flux de départ peut également contenir d'autres sels en tant qu'espèces ionisées.

Le flux de départ peut typiquement contenir, en pourcentages massiques, de 5 à 95 % d'eau, et notamment, toujours en pourcentages massiques : de 5 à 10 % d'eau ; ou de 10 à 15 % d'eau ; ou de 15 à 20 % d'eau; ou de 20 à 25 % d'eau; ou de 25 à 30 % d'eau; ou de 30 à 35 % d'eau ; ou de 35 à 40 % d'eau ; ou de 40 à 45 % d'eau ; ou de 45 à 50 % d'eau; ou de 50 à 55 % d'eau; ou de 55 à 60 % d'eau; ou de 60 à 65 % d'eau; ou de 65 à 70 % d'eau; ou de 70 à 75 % d'eau; ou de 75 à 80 % d'eau ; ou de 80 à 85 % d'eau ; ou de 85 à 90 % d'eau ; ou de 90 à 95 % d'eau.

Le flux de départ peut typiquement contenir, en pourcentages massiques, de 0,1 à 50 % de sulfate d'ammonium, et notamment, toujours en pourcentages massiques : de 0,1 à 1 % de sulfate d'ammonium ; ou de 1 à 5 % de sulfate d'ammonium ; ou de 5 à 10 % de sulfate d'ammonium ; ou de 10 à 15 % de sulfate d'ammonium ; ou de 15 à 20 % de sulfate d'ammonium ; ou de 20 à 25 % de sulfate d'ammonium ; ou de 25 à 30 % de sulfate d'ammonium ; ou de 30 à 35 % de sulfate d'ammonium ; ou de 35 à 40 % de sulfate d'ammonium ; ou de 40 à 45 % de sulfate d'ammonium ; ou de 45 à 50 % de sulfate d'ammonium.

Le flux de départ peut typiquement contenir, en pourcentages massiques, de 0,1 à 50 % d'acide 2-hydroxy-2-méthylpropionique, et notamment, toujours en pourcentages massiques : de 0,1 à 0,5 % d'acide 2-hydroxy-2-méthylpropionique ; ou de 0,5 à 1 % d'acide 2-hydroxy-2-méthylpropionique ; ou de 1 à 2 % d'acide 2-hydroxy-2-méthylpropionique ; ou de 2 à 3 % d'acide 2-hydroxy-2-méthylpropionique ; ou de 3 à 4 % d'acide 2-hydroxy-2-méthylpropionique ; ou de 4 à 5 % d'acide 2-hydroxy-2-méthylpropionique ; ou de 5 à 10 % d'acide 2-hydroxy-2-méthylpropionique ; ou de 10 à 15 % d'acide 2-hydroxy-2-méthylpropionique ; ou de 15 à 30 % d'acide 2-hydroxy-2-méthylpropionique ; ou de 30 à 50 % d'acide 2-hydroxy-2-méthylpropionique.

La purification chromatographique de l'invention repose de préférence sur un mécanisme d'exclusion d'ions, et non pas sur un mécanisme d'échange d'ions. Selon ce mécanisme d'exclusion d'ions, les sels et autres molécules chargées ont tendance à être exclues de la phase stationnaire, alors que les molécules neutres ont tendance à interagir avec la phase stationnaire et à être retenues par celle-ci.

La phase stationnaire reste de préférence essentiellement sous la même forme ionique pendant les étapes de charge et d'élution. De plus, le procédé de l'invention est de préférence dépourvu d'une étape de régénération de la phase stationnaire après l'élution.

Le pH du flux de départ peut être notamment de 0,5 à 6, de préférence de 1 à 5, de préférence encore de 1 à 4, lors de l'étape de charge.

Le procédé de l'invention peut comprendre une ou plusieurs étapes de pré-traitement en fonction de la variabilité du produit à traiter. Il peut s'agir de filtration, de séparation par échange d'ions ou encore de passage sur résine chélatante.

Alternativement, dans des modes de réalisation préférés, l'invention prévoit un unique passage sur une phase stationnaire, telle que décrite plus en détail ci-dessous. Ainsi, dans des modes de réalisation préférés, aucun passage sur une phase stationnaire qui est une résine anionique n'est prévu, en amont ou en aval.

La phase stationnaire utilisée peut notamment être une résine cationique. De préférence il s'agit d'une résine cationique forte, et de préférence encore d'une résine cationique forte (par exemple ayant des groupements fonctionnels sulfoniques) convertie sous forme NH₄⁺. Plusieurs résines cationiques de chromatographie peuvent être adaptées à cette séparation : Résine XA de Novasep, telle que XA2014/28K, les résines Dowex de Dow, Diaion de Mitsubishi, ou encore les résines cationiques de chromatographie de Lanxess, Finex ou Purolite.

De préférence, la phase stationnaire est sous forme de particules ayant une taille Dv50 de 200 à 350 µm.

Le terme Dv50 désigne le 50^{ème} centile de la distribution de taille des particules, c'est-à-dire que 50 % des particules ont une taille inférieure au Dv50 et 50 % ont une taille supérieure au Dv50. Il s'agit de la médiane de la distribution volumétrique des particules de résine.

La valeur du Dv50 peut être déterminée par granulométrie par diffraction laser. Dans certains cas, les particules individuelles peuvent avoir une tendance à l'agrégation, auquel cas il convient de déterminer leur taille par microscopie électronique, puisque la taille apparente mesurée par granulométrie par diffraction laser est alors plus importante que la taille particulaire réelle.

Le procédé de l'invention peut comprendre une étape préalable (avant le charge) d'équilibrage de la phase stationnaire, notamment : avec une solution contenant des ions ammonium (par exemple une solution de chlorure d'ammonium ayant un titre massique de 5 à 20 %, par exemple d'environ 10 %) ; ou bien, de préférence avec le flux de départ lui-même ayant par exemple un titre massique de 20 % environ. Dans un tel cas, une partie du flux de départ est consommée pour l'étape préalable d'équilibrage avant de procéder à la purification proprement dite

La température à laquelle le procédé est mis en oeuvre est de préférence de 20 à 85°C. Il est possible de prévoir des températures différentes pour les différentes étapes, mais de préférence, la température est sensiblement constante au cours du procédé.

La température peut notamment être de 20 à 25°C ; ou de 25 à 30°C ; ou de 30 à 35°C ; ou de 35 à 40°C ; ou de 40 à 45°C ; ou de 45 à 50°C ; ou de 50 à 55°C ; ou de 55 à 60°C ; ou de 60 à 65°C ; ou de 65 à 70°C ; ou de 70 à 75°C ; ou de 75 à 80°C ; ou de 80 à 85°C.

Une gamme de température de 50 à 80°C, et notamment de 60 à 70°C, peut être préférée.

Les étapes d'élution sont effectuées en faisant passer un éluant sur le lit de phase stationnaire. L'éluant peut être différent pour l'élution du raffinat ou de l'extrait. Un éluant ayant un gradient de composition peut également être utilisé. Toutefois, de préférence, le même éluant ayant une composition donnée est utilisé pour l'ensemble des élutions.

L'éluant peut être simplement de l'eau, de préférence de l'eau déminéralisée, sans ajustement du pH. L'eau déminéralisée peut typiquement avoir un pH de 5 à 7.

Alternativement, l'éluant peut être de l'eau (de préférence déminéralisée comme décrit ci-dessus) acidifiée, c'est-à-dire une solution aqueuse d'acide dans de l'eau (de préférence déminéralisée comme décrit ci-dessus). De préférence, l'acide utilisé est de l'acide sulfurique. D'autres possibilités sont : l'acide nitrique, l'acide chlorhydrique, l'acide citrique et les mélanges de ces acides.

La concentration de l'acide (notamment acide sulfurique) dans la solution aqueuse peut être notamment inférieure ou égale à 5 g/L, de préférence inférieure ou égale à 2 g/L, de préférence encore inférieure ou égale à 1 g/L, de préférence encore inférieure ou égale à 0,5 g/L et de préférence encore inférieure ou égale à 0,2 g/L. Des gammes de 0,0005 à 0,2 g/L, de préférence de 0,001 à 0,1 g/L, de préférence de 0,002 à 0,05 g/L, et de préférence encore de 0,005 à 0,02 g/L sont notamment appropriées.

Le pH de l'eau acidifiée utilisée comme éluant peut notamment être de 1 à 2 ; ou de 2 à 3 ; ou de 3 à 4 ; ou de 4 à 5 ; ou de 5 à 6. Un pH de 3,5 à 4,5, et notamment d'environ 4 peut être particulièrement souhaitable.

Le rapport massique de la quantité totale d'éluant consommé sur la quantité totale de flux de départ peut notamment être de 0,5 à 1 ; ou de 1 à 1,5 ; ou de 1,5 à 2 ; ou de 2 à 3 ; ou de 3 à 4 ; ou de 4 à 5 ; ou de 5 à 6 ; ou de 6 à 7 ; ou de 7 à 8 ; ou de 8 à 9 ; ou de 9 à 10 ; ou de 10 à 12 ; ou de 12 à 15 ; ou de 15 à 20. Des gammes de 2 à 8, notamment de 3 à 6, sont particulièrement préférées.

Lorsque le procédé est un procédé cyclique, dans lequel les étapes d'injection du flux de départ et d'élution sont effectuées de manière répétée dans une même colonne (appartenant typiquement à un système multicolonnes), les volumes ci-dessus sont les volumes constatés sur un cycle.

La vitesse opératoire moyenne de l'éluant à travers le lit de phase stationnaire, aussi bien pour l'élution de l'extrait que pour celle du raffinat, est de préférence supérieure à 1 m/h, ou supérieure à 2 m/h, ou supérieure à 4 m/h, et préférablement supérieure à 5 m/h. Cette vitesse opératoire moyenne correspond au débit volumique moyen divisé par la surface transversale moyenne du lit de phase stationnaire.

De préférence, la quantité massique totale de sels dans l'extrait est inférieure ou égale à 30 % de la quantité massique totale de sels dans le flux de départ, de préférence encore inférieure ou égale à 20 %, ou à 15 %, ou à 10 %, ou à 5 %. Des gammes de 1 à 15 % et de 1,5 à 10 % sont en particulier préférées.

De préférence, la quantité massique de sulfate d'ammonium dans l'extrait est inférieure ou égale à 30 % de la quantité massique de sulfate d'ammonium dans le flux de départ, de préférence encore inférieure ou égale à 20 %, ou à 15 %, ou à 10 %, ou à 5 %. Des gammes de 1 à 15 % et de 1,5 à 10 % sont en particulier préférées.

De préférence, la quantité massique d'acide 2-hydroxy-2-méthylpropionique dans l'extrait est supérieure ou égale à 80 % de la quantité massique d'acide 2-hydroxy-2-méthylpropionique dans le flux de départ, de préférence encore supérieure ou égale à 87%, de manière préférée supérieure ou égale à 90%.

Dans certains modes de réalisation, le pic d'élution des espèces ionisées, et notamment du sulfate d'ammonium (maximum de concentration des espèces ionisées dans le flux collecté) est compris entre 0,3 et 0,6 BV, de préférence entre 0,4 et 0,55 BV, et par exemple il peut être à approximativement 0,47 BV.

Dans certains modes de réalisation, le pic d'élution des espèces non ionisées et notamment de l'acide 2-hydroxy-2-méthylpropionique (maximum de concentration des espèces non ionisées dans le flux collecté) est compris entre 0,4 et 0,9 BV ; de préférence entre 0,5 et 0,7 BV, et par exemple il peut être à approximativement 0,67 BV.

L'unité BV (« *Bed Volume* ») correspond au volume du lit de phase stationnaire.

### Système chromatographique

Le procédé de l'invention peut être mis en oeuvre dans un système chromatographique à une colonne, ou de préférence dans un système chromatographique multicolonne. De préférence encore, le système chromatographique comprend de 4 à 10 colonnes.

De préférence, le procédé de l'invention est mis en oeuvre de façon continue.

De préférence, le procédé selon l'invention est un procédé chromatographique périodique à accumulation.

Par « *procédé à accumulation* », on entend un procédé chromatographique dans lequel l'injection du mélange à séparer (flux de départ) est intercalée ou additionnée à un profil de concentration non nulle passant de la sortie à l'entrée d'une colonne.

Des exemples de tels procédés à accumulation sont les procédés AMB, SMB, VariCol, Powerfeed, ModiCon, iSMB ou SSMB.

Le procédé à lit mobile simulé (ou SMB, pour « *simulated moving bed »)* est un procédé multi-colonne continu, l'injection de mélange à séparer étant effectuée sur l'ensemble d'un cycle.

Le procédé SMB peut être notamment un procédé SMB à quatre zones. Dans ce cas, le système comporte un ensemble de colonnes montées en série et en boucle fermée, la sortie d'une colonne étant reliée à une entrée de colonne suivante. Le système comprend au moins une ligne d'injection de mélange à séparer, une ligne de collecte de raffinat, une ligne d'injection d'éluant et une ligne de collecte d'extrait. Les lignes d'injection (de flux de départ et d'éluant) et les lignes de collecte de fractions se déplacent périodiquement et de manière synchrone (séquençage synchrone) au sein de la boucle dans la direction de l'écoulement du fluide circulant à travers la boucle. La durée entre deux décalages de l'ensemble des lignes d'injection et de collecte d'une colonne correspond à une période ; au bout d'un cycle tous les points sont revenus à leur position initiale, le système ayant un fonctionnement cyclique. Un cycle comporte autant de périodes que de colonnes.

Un système AMB (lit mobile réel, ou « *actual moving bed* ») présente un fonctionnement similaire à un système SMB. Toutefois, au lieu de déplacer les points d'injection du flux d'alimentation et de l'éluant, ainsi que des points de collecte, au moyen d'un système de vannes, un ensemble d'unités d'adsorption (colonnes) sont déplacées physiquement par rapport aux points d'alimentation et de collecte. A nouveau, le fonctionnement permet de simuler un lit mobile continu à contre-courant.

Le procédé selon l'invention peut être un procédé à injection continue du mélange à séparer (c'est-à-dire un procédé dans lequel l'injection du mélange à séparer est un flux continu). L'injection du mélange à séparer est alors effectuée pendant toute la durée du cycle. Le procédé selon l'invention peut également être un procédé à injection quasi-continue du mélange à séparer.

Alternativement, le procédé selon l'invention peut être un procédé dans lequel l'injection du mélange à séparer (flux de départ) est discontinue. Dans ces procédés, l'injection du mélange à séparer n'est pas faite sur l'ensemble d'un cycle mais pendant une durée totale inférieure à un cycle. On peut citer comme procédé à injection discontinue de mélange à séparer le procédé iSMB *(« improved simulated moving bed* » ou SMB amélioré en français), décrit dans les documents EP 0342629 et US 5,064,539, auxquels il est fait expressément référence. Dans ce procédé, dans une étape le système fonctionne en boucle fermée, sans injection ou collecte de produit.

Le procédé SMB séquentiel, ou SSMB (« *sequential simulated moving bed* ») est un autre exemple préféré. Un système SSMB découpe les introductions et collectes des flux en sous séquences appliquées de façons périodiques. Un système SSMB est par exemple décrit dans le document WO 2015/104464.

De préférence, le procédé selon l'invention est un procédé de type SSMB.

Lorsque le système chromatographique utilisé est multicolonne, et de préférence lorsque le procédé est à accumulation, le système chromatographique comprend de préférence des zones 1, 2, 3 et 4 : la zone 1 est située entre une ligne d'injection d'éluant et une ligne de collecte de l'extrait ; la zone 2 est située entre la ligne de collecte de l'extrait et une ligne d'injection du mélange à séparer ; la zone 3 est située entre la ligne d'injection du mélange à séparer et une ligne de collecte du raffinat ; et la zone 4 est située entre ladite ligne de collecte du raffinat et la ligne d'injection d'éluant.

Un exemple possible de système SSMB utilisable dans l'invention est représenté en référence à la **figure 1****.** Dans cet exemple, on utilise six cellules ou colonnes. Ce système peut être opéré selon un fonctionnement cyclique en quatre phases.
- Phase n°1 (partie A de la figure) : phase de boucle, durant laquelle on maintient une circulation continue en boucle fermée sur toutes les cellules placées en série, pour déplacer le volume interstitiel d'une cellule vers la suivante, sans injection d'éluant. L'homme de l'art observe que le volume de phase mobile déplacé dans cette phase contribue aux zones 1, 2, 3 et 4.
- Phase n°2 (partie B de la figure) : charge / injection de charge. La charge du flux (F) est injectée en tête de la quatrième cellule. Simultanément, on collecte un volume sensiblement identique de raffinat (R) en sortie de la cinquième cellule. Les cellules 4 et 5 constituent ici la zone 3. Les cellules 2 et 3 constituent la zone de séparation entre extrait et injection de charge. Elles constituent ici la zone 2. L'homme de l'art observe que le volume de phase mobile déplacé dans cette phase contribue à la zone 3.
- Phase n°3 (partie B de la figure) : élution de l'extrait. L'éluant (EL) est injecté sur la première cellule pour éluer l'extrait (EX), qui est collecté en volume sensiblement identique au bas de la première cellule. La cellule n°1 constitue ici la zone 1. L'homme de l'art observe que le volume de phase mobile déplacé dans cette phase contribue à la zone 1.
- Les phases n°2 et 3 sont de préférence opérées simultanément pour accroître la productivité du système.
- Phase n°4 (partie C de la figure) : élution du raffinat. L'éluant (EL) est injecté en tête de la première cellule, et le raffinat (R) est collecté en volume sensiblement identique sortie de la cinquième. La cellule n°6 est ici une cellule tampon permettant d'assurer la séparation entre la queue de l'extrait et la tête du raffinat. Elle constitue la zone 4. Cette zone peut être omise dans le cas où le degré de pureté et/ou le rendement recherché est relativement limité. L'homme de l'art observe que le volume de phase mobile déplacé dans cette phase contribue aux zones 1, 2 et 3.

Dans le cas d'un procédé multicolonne avec des zones identifiées entre les lignes d'entrées et sorties, le terme « *volume de phase mobile* » désigne le volume de fluide qui entre dans une zone. Ce fluide peut être différent de l'éluant au sens strict, mais il contribue au déplacement des produits dans chaque colonne de la zone. On parle ainsi de volume de phase mobile associé à chaque zone.

Dans le cas d'un procédé SSMB, le volume de zone 4 correspond au volume de phase mobile déplacé lors de la phase n°1 (partie 1 de la figure). Lorsque le procédé est un procédé cyclique, dans lequel les étapes d'injection du flux de départ et d'élution sont effectuées de manière répétée dans une même colonne (appartenant typiquement à un système multicolonnes), les volumes ci-dessus sont les volumes constatés sur un cycle. Dans le cas des procédés SSMB, SMB, VariCol, AMB et iSMB, le volume de phase mobile utilisée en zone 4 est de préférence compris entre 0,3 et 0,55 BV, de préférence encore entre 0,4 et 0,5 BV.

Ces phases sont opérées dans l'ordre dans un mode de réalisation préféré, de 1 à 4. Leur enchaînement constitue une séquence complète.

Chaque séquence (phases n°1 à 4) est répétée six fois en décalant les entrées et sorties de cellules par incrémentation du numéro de cellule, de la gauche vers la droite du système : la charge est ainsi injectée en haut de cellule n°1 en séquence n°1, puis en haut de cellule n°2 en séquence n°2, etc.

Un cycle de production complet est réalisé après achèvement des six séquences successives, quand le point d'injection de la charge, initialement en entrée de cellule n°1, revient de nouveau en entrée de cellule n°1.

Dans ce qui précède, on a donné une description du système SSMB en référence au cas où les cellules correspondent à des colonnes. Ceci n'est pas limitatif, et l'invention s'applique aussi aux systèmes dans lesquels les cellules, ou encore compartiments, sont des parties de colonne.

Par ailleurs, le nombre de colonnes présentes en zones 1, 2, 3 et 4 peut varier en fonction de la qualité de séparation désirée. On peut donc concevoir des systèmes du même type avec une cellule, deux cellules, trois cellules, quatre cellules et jusqu'à douze cellules ou plus.

### EXEMPLE

L'exemple suivant illustre l'invention sans la limiter.

Un mélange synthétique contenant 20 % massique de sulfate d'ammonium et 2,5 % massique d'acide 2-hydroxy-2-methylpropionique a été préparé.

Des tests ont été réalisés avec une résine cationique forte. Dans chaque test, 0,1 BV du mélange synthétique est injecté sur la résine puis élué.

La résine cationique est la suivante :
- Résine cationique Applexion^{®} XA 2014/28 K, préalablement convertie sous forme NH₄⁺ avec une solution de NH₄Cl à 10 % massique. La résine présente une capacité totale de 1,6 eq/L et une humidité de 55 à 59 % massique.

Deux tests ont été effectués en utilisant cette résine dans une colonne de 2,5 cm de diamètre et de 97,1 cm de hauteur. Les deux tests ont été effectués à une température de 65°C et avec un débit d'élution de 20 mL/min. Le volume d'injection du produit synthétique est de 5 mL. Dans l'essai n°1, l'éluant était de l'eau déminéralisée ; et dans l'essai n°2, l'éluant était de l'eau acidifiée avec de l'acide sulfurique à 0,005 g/L.

Des échantillons ont été prélevés tous les 0,05 BV entre 0,25 BV et 0,9 BV d'élution. Ces échantillons ont été analysés par conductimétrie avec un conductimètre S230 de METTLER TOLEDO, par chromatographie HPLC, afin de suivre l'élution des ions sulfate et ammonium ainsi que la concentration en acide 2-hydroxy-2-méthylpropionique, et par mesure de Brix.

Les caractéristiques de la chromatographie HPLC ionique (Dionex) sont les suivantes :
- Mesure des cations :
   ∘ Pré-colonne : IONPAC CG 12A 2mm ;
   ∘ Colonne : IONPAC CS 12A 2mm ;
   ∘ Phase mobile: acide hydroxyméthanesulfonique dégazé à 20 mM ;
   ∘ Débit : 0,25 mL/min.
- Mesure des anions :
   ∘ Pré-colonne : IONPAC AG 15 2mm ;
   ∘ Colonne : IONPAC AS 15 2mm ;
   ∘ Phase mobile : eau ultrapure reliée à un générateur KOH - gradient 11 à 71 mM ;
   ∘ Débit : 0,3 mL/min.

Les caractéristiques de la chromatographie HPLC utilisée pour mesurer la concentration de l'acide 2-hydroxy-2-methylpropionique sont les suivantes :
- Colonne HPLC : Aminex HPX 87H ;
- Phase mobile : 5 mM H₂SO₄ ;
- Détection : Refractive Index ;
- Température de la colonne : 60°C ;
- Température du détecteur : 55°C ;
- Volume d'injection : 20 µL ;
- Débit : 1 mL/min ;
- Temps d'analyse : 35 min ;
- Temps de rétention de l'acide 2-hydroxy-2-methylproprionique : 7,8 min ;
- Quantification : concentration exprimée en g/L.

Pour l'essai 1, les profils de séparation des différentes espèces présentes dans le produit synthétique permettent d'identifier un premier pic d'élution à 0,47 BV, correspondant au sulfate d'ammonium, et un second pic d'élution compris entre 0.62 et 0,67 BV correspondant à l'acide 2-hydroxy-2-methylpropionique.

Il est également constaté un taux d'élimination du sulfate d'ammonium de 98,5 %, c'est-à-dire que 98,5 % du sulfate d'ammonium initialement présent dans le produit synthétique est élué dans le raffinat.

Il est également constaté un taux de récupération de l'acide 2-hydroxy-2-methylpropionique de 87 %, c'est-à-dire que 87 % de l'acide 2-hydroxy-2-methylpropionique initialement présents dans le produit synthétique est élué dans l'extrait.

Pour l'essai 2, les profils de séparation des différentes espèces présentes dans le produit synthétique permettent d'identifier un premier pic d'élution à 0,47 BV, correspondant au sulfate d'ammonium, et un second pic d'élution compris à 0,67 BV correspondant à l'acide 2-hydroxy-2-methylpropionique.

Il est également constaté un taux d'élimination du sulfate d'ammonium de 98,5 %, c'est-à-dire que 98,5 % du sulfate d'ammonium initialement présent dans le produit synthétique est élué dans le raffinat.

Il est également constaté un taux de récupération de l'acide 2-hydroxy-2-methylpropionique de 91 %, c'est-à-dire que 91 % de l'acide 2-hydroxy-2-methylpropionique initialement présents dans le produit synthétique est élué dans l'extrait.

Ces essais montrent que la séparation est possible et naturellement les valeurs de volumes de rétentions peuvent varier d'un essai à l'autre ou d'une phase stationnaire cationique à l'autre. Toutefois la différence de volumes de rétention est suffisante pour effectuer une purification à l'échelle industrielle.

A partir de cet exemple, et à l'aide d'un logiciel de simulation chromatographique, en utilisant par exemple les méthodes décrites dans le manuel Preparative Chromatography of Fine Chemicals and Pharmaceutical Agents, Henner Schmidt-Traub, Wiley-VCH, ISBN-13 978-3-527-30643-5, il est possible de déduire un rapport entre la quantité d'éluant et la quantité de produit injectée dans la colonne de chromatographie compris entre 1,5 et 5,4. Ce rapport volumique évolue en fonction de la longueur et du nombre de colonnes ainsi que de la composition de la charge à traiter, si par exemple la teneur en acide 2-hydroxy-2-méthylpropionique est comprise entre 5 et 10 % ou entre 1 et 5 %. Les calculs ont permis de montrer que la purification peut être effectuée sur une grande gamme de compositions initiales.

Le procédé selon l'invention peut avantageusement être effectué dans un système SSMB de quatre à six colonnes avec une hauteur de colonne comprise entre 1,5 et 2,5 m, selon le type d'éluant utilisé et la vitesse opératoire d'élution qui sera supérieure à 1 m/h, ou supérieure à 2 m/h, ou supérieure à 4 m/h, et préférablement supérieure à 5 m/h. Il est toutefois déterminé qu'il est possible d'utiliser de longueur de colonne par exemple de 0,5 à 1 m si l'on utilise un plus grand nombre de colonnes, par exemple 12 ou 24. Ces calculs montrent qu'un diamètre de colonne supérieur à deux mètres permet de traiter un flux de plusieurs mètres cubes par heure. Un élément important obtenu lors de ces calculs est la contrainte sur le volume de zone 4 à utiliser afin de déminéraliser l'acide 2-hydroxy-2-méthylpropionique, qui est compris entre 0,3 et 0,55 BV, de préférence entre 0,4 et 0,5 BV.

## Revendications

1. Procédé de purification chromatographique d'un flux de départ contenant du sulfate d'ammonium et de l'acide 2-hydroxy-2-méthylpropionique, comprenant :
- le passage du flux de départ sur un lit de phase stationnaire ;
- l'élution d'un raffinat enrichi en sulfate d'ammonium et appauvri en acide 2-hydroxy-2-méthylpropionique ; et
- l'élution d'un extrait enrichi en acide 2-hydroxy-2-méthylpropionique et appauvri en sulfate d'ammonium.

2. Procédé selon la revendication 1, dans lequel la purification est effectuée par chromatographie d'exclusion d'ions.

3. Procédé selon l'une des revendications 1 à 2, dans lequel la phase stationnaire est une résine cationique, de préférence une résine cationique forte, et de préférence encore une résine cationique forte sous forme de contre-ion NH₄⁺.

4. Procédé selon l'une des revendications 1 à 3, dans lequel la phase stationnaire est constituée de particules ayant une taille Dv50 comprise entre 200 et 350 µm.

5. Procédé selon l'une des revendications 1 à 4, dans lequel la phase stationnaire est préalablement équilibrée avec une solution contenant des ions ammonium, et de préférence avec le flux de départ.

6. Procédé selon l'une des revendications 1 à 5, dans lequel le flux de départ est à un pH de 1 à 4 lors de son passage sur le lit de phase stationnaire.

7. Procédé selon l'une des revendications 1 à 6, dans lequel on fait passer un éluant sur le lit de phase stationnaire pour l'élution du raffinat et l'élution de l'extrait, ledit éluant étant de préférence de l'eau déminéralisée ou de l'eau déminéralisée acidifiée, notamment à un pH de 3 à 5, l'eau déminéralisée acidifiée ayant de préférence une teneur en acide de 0,0005 à 5 g/L, de préférence encore de 0,005 à 0,02 g/L.

8. Procédé selon l'une des revendications 1 à 7, qui est mis en oeuvre à une température de 20 à 85°C, de préférence de 50 à 80°C.

9. Procédé selon l'une des revendications 1 à 8, qui est mis en oeuvre sur un système chromatographique multicolonnes, de préférence un système à lit mobile simulé, et de préférence encore un système à lit mobile simulé séquentiel.

10. Procédé selon la revendications 9, dans lequel le système chromatographique comprend une zone 4 située entre une ligne de collecte de raffinat et une ligne d'injection d'éluant, la zone 4 étant traversée par un volume de phase mobile compris entre 0,3 et 0,55 BV, de préférence entre 0,4 et 0,5 BV.

11. Procédé selon la revendication 9 ou 10, dans lequel le système chromatographique multicolonnes comprend moins de 12 colonnes, ou moins de 8 colonnes, et de préférence de 4 à 6 colonnes.

12. Procédé selon les revendications 9 à 11, dans lequel le système chromatographique multicolonnes comprend des colonnes ayant un diamètre supérieur ou égal à 2 mètres.

13. Procédé selon les revendications 9 à 12, dans lequel le système chromatographique multicolonnes comprend des colonnes ayant une hauteur comprise entre 1 et 2,5 mètres.

14. Procédé selon l'une des revendications 1 à 13, qui est mis en oeuvre avec une vitesse opératoire moyenne d'élution supérieure à 1 m/h, ou supérieure à 2 m/h, ou supérieure à 4 m/h, et préférablement supérieure à 5 m/h.

15. Procédé selon l'une des revendications 1 à 14, dans lequel le flux de départ est issu d'un procédé de production de méthacrylate de méthyle, ledit procédé de production de méthacrylate de méthyle comprenant de préférence :
- la préparation de sulfate de méthacrylamide par réaction de la cyanhydrine d'acétone avec de l'acide sulfurique ;
- la préparation de méthacrylate de méthyle par réaction dudit sulfate de méthacrylamide avec un mélange d'eau et de méthanol ;
- la collecte d'un effluent acide ;
- la neutralisation de l'effluent acide avec de l'ammoniac ;
- la cristallisation de l'effluent acide neutralisé pour obtenir d'une part des cristaux de sulfate d'ammonium et d'autre part une purge de cristallisation, cette purge de cristallisation fournissant ledit flux de départ.

## Patentansprüche

1. Verfahren zur chromatographischen Reinigung eines Ausgangsstroms, der Ammoniumsulfat und 2-Hydroxy-2-methylpropionsäure enthält, umfassend :
- Leiten des Ausgangsstroms über ein Bett aus einer stationären Phase ;
- Elution eines Raffinats, das mit Ammoniumsulfat angereichert und an 2-Hydroxy-2-methylpropionsäure verarmt ist; und
- die Elution eines an 2-Hydroxy-2-methylpropionsäure angereicherten und an Ammoniumsulfat abgereicherten Extrakts.

2. Verfahren nach Anspruch 1, wobei die Reinigung durch Ionenausschlusschromatographie durchgeführt wird.

3. Verfahren nach einem der Ansprüche 1 bis 2, wobei die stationäre Phase ein kationisches Harz ist, vorzugsweise ein starkes kationisches Harz, und noch bevorzugter ein starkes kationisches Harz in Form des Gegenions NH₄⁺.

4. Verfahren nach einem der Ansprüche 1 bis 3, wobei die stationäre Phase aus Partikeln mit einer Dv50-Größe zwischen 200 und 350 µm besteht.

5. Verfahren nach einem der Ansprüche 1 bis 4, wobei die stationäre Phase zuvor mit einer Lösung, die Ammoniumionen enthält, und vorzugsweise mit dem Ausgangsstrom ausgeglichen wird.

6. Verfahren nach einem der Ansprüche 1 bis 5, wobei der Ausgangsstrom bei seinem Durchgang durch das Bett der stationären Phase einen pH-Wert von 1 bis 4 aufweist.

7. Verfahren nach einem der Ansprüche 1 bis 6, bei dem zur Elution des Raffinats und zur Elution des Extrakts ein Elutionsmittel über das Bett der stationären Phase geleitet wird, wobei das Elutionsmittel vorzugsweise entmineralisiertes Wasser oder angesäuertes entmineralisiertes Wasser ist, insbesondere bei einem pH-Wert von 3 bis 5, wobei das angesäuerte entmineralisierte Wasser vorzugsweise einen Säuregehalt von 0,0005 bis 5 g/L, weiter bevorzugt 0,005 bis 0,02 g/L aufweist.

8. Verfahren nach einem der Ansprüche 1 bis 7, das bei einer Temperatur von 20 bis 85 °C, vorzugsweise von 50 bis 80 °C, durchgeführt wird.

9. Verfahren nach einem der Ansprüche 1 bis 8, das auf einem chromatographischen Mehrsäulensystem, vorzugsweise einem simulierten Wanderbettsystem und noch bevorzugter einem sequenziellen simulierten Wanderbettsystem, durchgeführt wird.

10. Verfahren nach Anspruch 9, wobei das Chromatographiesystem eine Zone 4 umfasst, die sich zwischen einer Raffinatsammelleitung und einer Elutionsmittelinjektionsleitung befindet, wobei die Zone 4 von einem Volumen der mobilen Phase zwischen 0,3 und 0,55 BV, vorzugsweise zwischen 0,4 und 0,5 BV, durchströmt wird.

11. Verfahren nach Anspruch 9 oder 10, wobei das chromatographische Mehrsäulensystem weniger als 12 Säulen oder weniger als 8 Säulen und vorzugsweise 4 bis 6 Säulen umfasst.

12. Verfahren nach den Ansprüchen 9 bis 11, wobei das chromatographische Mehrsäulensystem Säulen mit einem Durchmesser von 2 m oder mehr umfasst.

13. Verfahren nach Anspruch 9 bis 12, wobei das chromatografische Mehrsäulensystem Säulen mit einer Höhe zwischen 1 und 2,5 Metern umfasst.

14. Verfahren nach einem der Ansprüche 1 bis 13, das mit einer durchschnittlichen operativen Elutionsgeschwindigkeit von mehr als 1 m/h oder mehr als 2 m/h oder mehr als 4 m/h und vorzugsweise mehr als 5 m/h durchgeführt wird.

15. Verfahren nach einem der Ansprüche 1 bis 14, wobei der Ausgangsstrom aus einem Verfahren zur Herstellung von Methylmethacrylat stammt, wobei das Verfahren zur Herstellung von Methylmethacrylat vorzugsweise Folgendes umfasst:
- die Herstellung von Methacrylamidsulfat durch Umsetzung von Acetoncyanhydrin mit Schwefelsäure;
- die Herstellung von Methylmethacrylat durch die Reaktion des genannten Methacrylamidsulfats mit einer Mischung aus Wasser und Methanol;
- das Sammeln eines sauren Abflusses;
- Neutralisieren des sauren Abflusses mit Ammoniak;
- Kristallisieren des neutralisierten sauren Abflusses, um einerseits Ammoniumsulfatkristalle und andererseits eine Kristallisationsspülung zu erhalten, wobei diese Kristallisationsspülung den genannten Ausgangsstrom liefert.

## Claims

1. A method for chromatographically purifying a starting stream containing ammonium sulphate and 2-hydroxy-2-methylpropionic acid, comprising the steps of:
- passing the starting stream through a stationary-phase bed;
- eluting a raffinate enriched in ammonium sulphate and depleted in 2-hydroxy-2-methylpropionic acid; and
- eluting an extract enriched in 2-hydroxy-2-methylpropionic acid and depleted in ammonium sulphate.

2. The method according to claim 1, wherein the purifying step is performed by ion-exclusion chromatography.

3. The method according to any one of claims 1 to 2, wherein the stationary phase is a cationic resin, preferably a strong cationic resin, more preferably a strong cationic resin in the form of NH₄⁺ counter ion.

4. The method according to any one of claims 1 to 3, wherein the stationary phase consists of particles having a Dv50 size of between 200 and 350 µm.

5. The method according to any one of claims 1 to 4, wherein the stationary phase is pre-equilibrated with a solution containing ammonium ions, and preferably the starting stream.

6. The method according to any one of claims 1 to 5, wherein the starting stream is at a pH of 1 to 4 as it passes through the stationary-phase bed.

7. The method according to any one of claims 1 to 6, wherein an eluent is passed through the stationary-phase bed to elute the raffinate and elute the extract, wherein said eluent is preferably demineralised water or acidified demineralised water, in particular at a pH of 3 to 5, wherein the acidified demineralised water preferably has an acid content of 0.0005 to 5 g/L, more preferably 0.005 to 0.02 g/L.

8. The method according to any one of claims 1 to 7, which is performed at a temperature of 20 to 85 °C, preferably 50 to 80 °C.

9. The method according to any one of claims 1 to 8, which is performed on a multi-column chromatography system, preferably a simulated moving bed system, and more preferably a sequential simulated moving bed system.

10. The method according to claim 9, wherein the chromatography system comprises a zone 4 located between a raffinate collection line and an eluent injection line, wherein the zone 4 is traversed by a volume of mobile phase of between 0.3 and 0.55 BV, preferably between 0.4 and 0.5 BV.

11. The method according to claim 9 or 10, wherein the multi-column chromatography system comprises less than 12 columns or less than 8 columns and preferably 4 to 6 columns.

12. The method according to claims 9 to 11, wherein the multi-column chromatography system comprises columns having a diameter of greater than or equal to 2 metres.

13. The method according to claims 9 to 12, wherein the multi-column chromatography system comprises columns having a height of between 1 and 2.5 metres.

14. The method according to any one of claims 1 to 13, which is performed at a mean elution operating speed of greater than 1 m/h or greater than 2 m/h or greater than 4 m/h and preferably greater than 5 m/h.

15. The method according to any one of claims 1 to 14, wherein the starting stream is derived from a methyl methacrylate production process, wherein said methyl methacrylate production process preferably comprises the steps of:
- reacting acetone cyanohydrin and sulphuric acid to produce methacrylamide sulphate;
- reacting said methacrylamide sulphate and a mixture of water and methanol to produce methyl methacrylate;
- collecting an acidic effluent;
- neutralising the acidic effluent using ammonia;
- crystallising the neutralised acidic effluent to obtain ammonium sulphate crystals on the one hand and a crystallisation purge on the other, wherein this crystallisation purge provides the starting stream.
